Europäisches Patentamt

European Patent Office

Office européen des brevets

(11) Publication number: **0 288 621 B1**

# EUROPEAN PATENT SPECIFICATION

(45) Date of publication of patent specification: **10.03.93**    (51) Int. Cl.⁵: **C12Q 1/04**, C12M 1/12

(21) Application number: **87303715.4**

(22) Date of filing: **27.04.87**

(54) **Method and apparatus for detection and quantitation of bacteria.**

(43) Date of publication of application:
**02.11.88 Bulletin 88/44**

(45) Publication of the grant of the patent:
**10.03.93 Bulletin 93/10**

(84) Designated Contracting States:
**DE FR GB IT**

(56) References cited:
**EP-A- 0 122 581**
**GB-A- 1 441 022**
**US-A- 4 225 669**
**US-A- 4 336 337**
**US-A- 4 639 421**

(73) Proprietor: **Texas BioResource Corporation**
**P.O. Box 91627**
**Austin Texas 78709-1627(US)**

(72) Inventor: **Longoria, Claude C.**
**2927 Field Line Drive**
**Sugar Land, TX 77479(US)**

(74) Representative: **Adams, William Gordon et al**
**RAWORTH, MOSS & COOK 36 Sydenham**
**Road**
**Croydon Surrey CR0 2EF (GB)**

**Description**

Field of the Invention

This invention relates generally to testing procedures for bacteria in fluid samples such as human body fluids and more particularly to a method and apparatus for efficient, less cost, rapid testing procedures for identifying the presence and concentration of gram negative and gram positive bacteria strains in fluid samples.

Background of the Invention

A need exists for a method to rapidly detect bacteria in urine and various other fluid samples.

Urine specimens are the major work load of the diagnostic laboratory. The most common urological disease is urinary-tract infections in children, pregnant women, diabetics and geriatric patients, (bacteriuria). In hospitals, bacteriuria is the predominant form of nosocomial infection. In view of the frequent asymptomatic urinary-tract infections bacteriuria tests must be sufficiently simple and economical to permit routine testing. A need, therefore, exists for simple, rapid, inexpensive screening tests to facilitate diagnosis and ensure prompt treatment of bacteriuric patients.

The conventional method for detection of urinary-tract infections is by inoculation of urine onto agar culture plates and incubation of these plates at optimal temperatures (usually 37 degrees C) for 24-48 hours; bacteria are detected by formation of colonies on the agar surface. Fastidious bacteria may not grow on conventional culture media, and a variety of agar media may be required to detect these bacteria. This procedure is time consuming and expensive.

Although staining techniques, such as the Gram Stain Method, are known in clinical microbiology, light microscopy is required, and the procedures are time consuming and require a skilled microbiologist. Other prior art processes involve staining of bacteria followed by concentration by centrifugation or filtration. These processes require chelating agents and positively-charged small pore filters, and suffer clogging and interference from anionic pigments, blood and other substances which may be present in urine.

In US-A-4 336 377 bacteria is detected by a test substrate, including a semi-permeable membrane, which retains bacteria on the membrane and which has net positive surface charge to permit colouring of bacteria thereon with a cationic dye, without the membrane adsorbing thereon substantial amounts of said dye. In US-A-4 225 669 both gram-negative and gram-positive bacteria are detected by contacting the bacteria sample with a chelating agent and a basic dye, operative at a pH above 7.0. The stained bacteria can be concentrated by filtration, centrifugation and the like, whereby the gradation of colour of the stained concentrated bacteria corresponds to the number of bacteria. Differentiation of gram-negative from gram-positive bacteria can be accomplished by treating the stained bacteria with an organic acid wash having a pH of 2.5 to 2.6. This wash completely decolorizes only gram-positive bacteria. In EP-A-0 122 581 bacteria can be isolated from blood by mixing blood to be examined with a liquid containing a hemolytic agent and an anticoagulant. The resultant mixture is filtered through a filter having a pore size that does not allow passage therethrough of the bacteria to be separated. Nutrients are then supplied to the bacteria on the filter through the mixture so as to immediately culture the bacteria on the filter.

It has now been unexpectedly discovered that both gram negative and gram positive bacteria can be stained for simple, rapid detection by means of the composition of the present invention. Concentrated bacteria stained with the composition described herein are readily visible and can thus be rapidly detected without resorting to light microscopic examination by specially trained personnel, or the use of expensive, perishable agar cultures. The present invention allows the efficient detection of bacteria while reducing clogging and pigment interference common to prior art methods. Furthermore, it was discovered through tests that inexpensive, simple, and rapid quantitative analysis of bacteria is possible employing the present staining composition.

Summary of the Invention

The method of the invention takes use of a composition for staining both gram negative and gram positive bacteria. The composition comprises an inorganic acid diluent, a dye soluble at a pH of 8 to 12 which preferentially stains bacteria, a washing reagent composed of an inorganic acid, and a filter paper or glass filter of a net negative charge. Bacteria are electrostatically immobilized and concentrated on the filter and are stained with the dye solution. The free dye is then removed with the washing reagent. The bacteria, which retain the dye, become visible and thus may be detected and quantitated by comparing the color

2

intensity of the filter surface (due to the stained bacteria) to a nomograph or other calibrated standard based on color intensities obtained using known amounts of bacteria. The total test time is usually less than one minute.

It is therefore a primary feature of the present invention to provide a novel method and apparatus for rapid detection and quantitation of bacteria to thus enable rapid initiation of treatment of patients for bacterial infections.

It is also a feature of this invention to provide novel method and apparatus for bacteria detection wherein a number of tests may be conducted simultaneously for detection and quantitation of bacteria to thus minimize the time period for detection of bacteria that might be present in samples of body fluid.

It is another feature of this invention to provide novel method and apparatus for identifying the presence and concentrations of both gram negative and gram positive bacteria strains in fluid samples.

Among the several features of this invention is contemplated the provision of a novel method and apparatus for bacteria detection through employment of a dye for staining of the bacteria together with a filter composed of paper, glass, or any other suitable material having a net negative charge for electrostatically immobilizing and concentrating the bacteria on the filter prior to staining.

It is another feature of this invention to provide a novel method and apparatus for quantifying the concentration of bacteria in a fluid sample by means of visible comparison of the color intensity of stained bacteria on the filter surface with a nomograph or other calibrated standard based on color intensities obtained using known concentrations of bacteria.

Brief Description of the Drawings

So that the manner in which the above recited features, advantages and objects of the present invention are attained and can be understood in detail, more particular description of the invention, briefly summarized above, may be had by reference to the embodiments thereof which are illustrated in the appended drawings.

It is to be noted, however, that the appended drawings illustrate only typical embodiments of this invention and are therefore not to be considered limiting of its scope, for the invention may admit to other equally effective embodiments.

Preferred embodiments of this invention have been chosen for purposes of illustration and description and are shown in the accompanying drawings forming a part of this specification wherein:

Fig. 1 is a plan view of filter apparatus constructed in accordance with the present invention.

Fig. 2 is a sectional view taken along line 2-2 of Fig. 1 and showing the various parts of the filter in exploded relation.

Fig. 3 is a sectional view showing the assembled filter apparatus of Figs. 1 and 2.

Fig. 4 is a plan view of filter apparatus representing an alternative embodiment of this invention and providing for simultaneous multiple testing of a body fluid sample.

Fig. 5 is a sectional view taken along line 5-5 of Fig. 4 which illustrates one position of the filter apparatus of Fig. 4.

Fig. 6 is a sectional view similar to that of Fig. 5 showing another position of the filter apparatus.

Detailed Description of the Invention

The invention relates to a process for concentrating, immobilizing and staining bacteria contained within a fluid sample, comprising:

(a) diluting the fluid sample;

(b) adsorbing the bacteria in the fluid sample to a designated portion of a filter;

(c) staining said adsorbed bacteria at a basic pH with a staining dye operative at a basic pH; and

(d) diffusing free staining dye from said stained adsorbed bacteria, characterized in that in step (a) the fluid sample is diluted with an inorganic acid; in step (b) the bacteria in the fluid sample is electrostatically adsorbed to the filter having a net negative charge and in step (d) the free staining dye is diffused from said stained electrostatically adsorbed bacteria to a region of said filter beyond said designated portion, leaving stained bacteria in said designated region for comparative quantitative analysis.

The invention relates also to an apparatus for concentrating, immobilizing and staining bacteria from a fluid sample according to the process of any one of Claims 1 to 6, comprising:

(a) disk means (1, 10) having upper and lower surfaces and an orifice (3);

(b) filter means (5);

3

(c) means (6, 8) retaining said filter means (5) against said lower surface of said disk means (1, 10) and means for color comparison indicating quantitation of said stained bacteria, characterized in that the disk means in (a) form a sample reservoir means (2) extending from said upper surface and opening through said orifice for conducting, in use, the fluid sample to said lower surface; the filter means in (b) has a net negative charge; and in (c) a portion of said filter means (5) is exposed to said sample reservoir means (2) at said orifice, said filter means (5) extending radially from said opening a sufficient distance in order to, in use, permit radial diffusion of free dye from said portion of the filter means (5) exposed at said orifice and leave concentrated and stained bacteria from said fluid sample at said exposed portion of said filter means (5).

Since the color intensity of the filter is proportional to the number of bacteria in the fluid sample, quantitative analysis of bacteria may be accomplished by comparing the color intensity of the stained bacteria on the filter surface to a known standard.

The method and apparatus of the invention have particular application to the detection and quantitation of bacteria in urine. By means of this invention, rapid and economical detection of urinary tract infection is possible.

More particularly, it has been discovered that dyes soluble at pH 8 to 12 which stain bacteria will do so in the absence of chelating agents. Bacteria are stained by contacting these organisms, which are electrostatically adsorbed to negatively charged filter surfaces, with a protein staining dye at about pH 8 to 12. Any dye capable of staining bacteria which is soluble at about pH 8 to 12 may be employed. However, preferable dyes are Safranin-O or Basic Fuchsin.

Bacteria stained in accordance with the present invention may be readily detected if present at a concentration of 50,000 or higher per milliliter, or at lower amounts if concentrated. Stained bacteria which are dispersed in solution will, upon concentration, be readily visible. When concentrated bacteria at sufficient concentration are stained without dispersion, the presence of bacteria is immediately manifest.

Sedimentation and entrapment of bacteria by filtration are examples of effective means for concentrating bacteria. In this current invention, bacteria in urine (their isoelectric charge having been modified by an acid diluent) are adsorbed to a negatively charged filter surface. As the urine-diluent solution diffuses over a wide area, the bacteria therein electrostatically adsorb to the filter at the site where the urine is deposited, providing an effective method for concentrating bacteria. In accordance with the method of this invention, the pores of the filter are not required to be smaller than the bacteria under test.

Filter surfaces which have a net negative charge rather than a net positive charge must be employed. The diluent indicated in this application will reverse the isoelectric charge of negatively charged bacteria, altering the same to a positive charge. Thus, the bacteria in the urine-diluent solution will be immobilized electrostatically by adsorption to the filter matrix whereas free dye, urine pigments and other urinary compounds will diffuse away from the restricted area site of the adsorbed bacteria allowing detection of the stained organisms.

Dye used to stain bacteria at the site of the deposit of the urine-diluent mixture is diffused away from the immobilized stained bacteria adsorbed to the filter. Addition of the washing reagent at the site at which the urine-diluent solution was deposited enhances the diffusion of the free dye from this site without removing the stain from the immobilized bacteria.

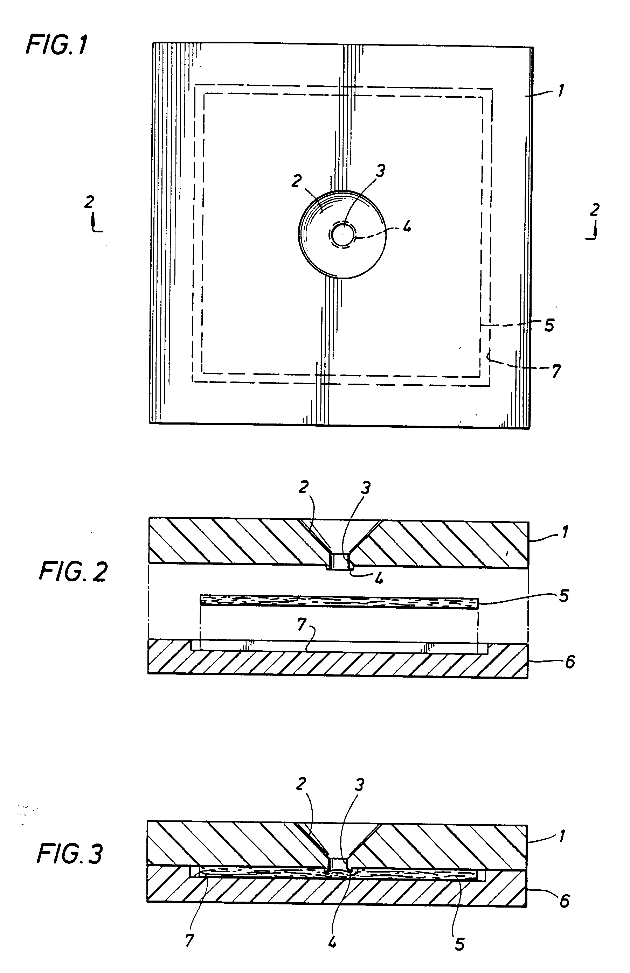

Referring now to the drawings, a preferred embodiment of the invention is illustrated in Fig. 1 and consists of a flat, round or square, rigid or semi-rigid disk 1. This disk may be machined, molded or thermoformed from a variety of materials, e.g., polystyrene. The disk preferably measures about one to four inches in diameter or width and features a conical or round depression or reservoir 2 at or near the center of the disk. Located at the bottom of the reservoir the disk defines a short bore forming a small diameter hole or orifice 3 which extends through the entire thickness of the disk. The orifice diameter may be in the range of from about 1,5 mm to about 6,35 mm (about 0.06 inch to about 0.25 inch).

Though it is not an absolute requirement for performance of the procedure, the disk may feature an orifice lip 4 protruding from the lower surface around the orifice. This orifice lip allows for more efficient flow of fluids into a precise location on the filter paper pad and serves to inhibit the spreading of fluid on the upper surface of the filter pad. The filter pad 5 is positioned adjacent to the lower surface of the disk so that the filter pad is in contact with the orifice or orifice lip. The filter pad may be composed of cellulose, fiberglass or other suitable filter material and exhibits a net negative electrostatic charge.

The position of the filter pad is maintained by "sandwiching" the pad between the disk and a lower filter retainer plate 6. The lower plate may be machined, molded or thermoformed, and features a square or round depression 7 slightly larger in size than the filter pad. The lower plate may be attached to the disk to secure the filter pad in place. This attachment may be by any suitable means such as by an adhesive, solvent welding, or sonic welding. Alternatively, the disk and lower plate may be designed to hinge, snap or screw together to house the filter pad.

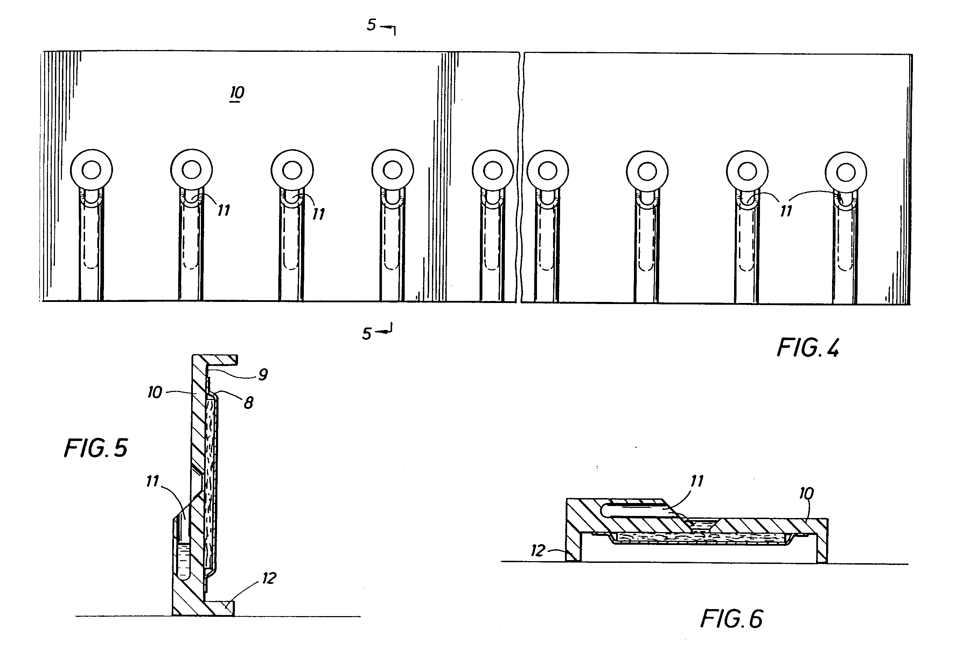

In another embodiment of the invention, as evidenced by Figs. 4-6. an adhesive label or tape 8 is applied to the lower surface 9 of the disk or plate 10 to secure the filter pad, thus eliminating the need for a lower filter retainer plate.

The embodiments described above are useful as a single use disposable, portable unit for detection of bacteria and have particular application for detection of bacteria in fluids, e.g., urine, in situations where conventional methods for bacteria detection are cumbersome or unfeasible, e.g., over-the-counter for consumer home use, physicians' offices, screening clinics, etc.

The disk may be formed to define a reaction well 11 which extends transversely from the tapered reservoir as illustrated in Fig. 4. When the device is placed in a vertical position, with its base 12 resting on any flat horizontal surface, as shown in Fig. 5, the reaction well 11 is positioned to accept and retain sample fluid. When the device is subsequently placed in a horizontal or near horizontal position as shown in Fig. 6 by 90 degrees clockwise rotation from the position shown in Fig. 5, the fluid flows out of the reaction well and into the reservoir. The fluid then travels through the orifice and upon contact with the filter pad, is absorbed into the pad. This feature is especially useful in tests requiring incubation or reaction of a bacterial suspension with a chemical substance prior to performance of the bacterial quantitation test. In particular, a test for determination of antibiotic susceptibility may be performed by incubating a bacterial suspension with an appropriate antibiotic for a period of time during which, if the organism is resistant to the antibiotic, growth of the organism will occur. After the incubation period, the device is placed in a horizontal position to allow the antibiotic-treated bacterial suspension to flow to the reservoir and filter pad, thus allowing for quantitation with the staining composition of the current invention.

A disposable multiple test device is illustrated in Fig. 4 which may be made by molding or thermoforming a device featuring two or more units of the embodiments described above in connection with Fig. 4. This multiple test device has particular application in a test for determination of antibiotic susceptibility. The appropriate antibiotics may be pre-loaded into the reaction well thus allowing for simple test set-up and multiple determination of antibiotic susceptibility.

Although specific embodiments of the invention have been shown and described, it is understood that other embodiments adaptations and modifications may be utilized without departing from the true spirit and scope of the invention. The embodiments, composition and methods described herein may be similarly applied to the staining and detection of bacteria in other fluids, such as culture media, blood, spinal fluids, food washings, milk and water, as well as to staining bacteria from such fluids which have been deposited on filter surfaces.

The minimum quantity of bacteria which can be detected by this staining procedure varies somewhat with the condition and growth phase of the bacterial culture. In general, actively growing, viable bacteria are strongly detected at levels of about 100,000/ml. Non-viable organisms or organisms in lag phase stain less intensely and thus higher levels of bacteria are required for detection.

The following examples are illustrative of the invention and are not to be taken in a limiting sense.

Example 1

Two drops of urine (using a conventional dropping pipette) from a known positive bacteriuric patient was added to 8 drops of acid diluent, pH 1.5 HCl in a test tube. Four drops of the mixture was added to the center of a negatively charged filter. Three drops of Safranin-O dye at 1:1000 in pH 10 carbonate-borate-hydroxyl buffer was added to the filter surface at the site of inoculation of the urine-diluent mixture. Three drops of washing reagent, pH 2.5 HCl was added to the same site to allow diffusion of free dye away from the site of inoculation. Three additional drops of washing reagent was used as a second rinse. The results manifested a 3-4 mm diameter intensely stained filter surface at the site of inoculation with a peripheral ring of free dye about 25 mm from the site of the stained bacteria. Such a result is indicative of a positive bacteriuria test. Comparison of the intensity of the color of the stained bacteria to a known amount of bacteria added to urine indicated about 1,000,000 bacteria per ml urine. Plating of the sample on agar culture verified these results.

Example 2

Two drops of urine (using a conventional dropping pipette) from a healthy volunteer (free of urinary-tract infection by conventional assay) was added to eight drops of acid diluent, pH 1.5 HCl in a test tube. Four drops of the mixture was added to the center of a negatively charged filter. The remainder of this experiment was conducted exactly as described in Example 1 above. Upon observation, the filter surface at the site at which the urine-diluent solution was deposited was completely white and the free dye ringed the filter at about 25 mm from the site of the inoculation. Such a result is indicative of a negative bacteriuria test. This result was verified by plating of the sample on agar culture media.

Example 3

Employing the procedure set forth in Examples 1 and 2, and the preferred embodiment illustrated in Fig. 1, experiments were conducted with a variety of organisms using Safranin-O as a model dye at pH 10 in carbonate-borate-hydroxyl buffer. The results were as follows:

| Test Organisms 100,000 Bacteria/ml | Intensity of Stain* On Filter Surface |
|---|---|
| Normal urine, no bacteria | 0 |
| E. coli | ++ |
| S. aureus | ++ |
| Proteus vulgaris | ++ |
| Pseudomonas | ++ |
| Group A Strep. | ++ |
| Group D Strep. | ++ |
| Klebsiella pn. | ++ |

*Site of inoculation of sample of urine-diluent onto the filter surface. Scoring of color intensity: 0 = white, no color; += light pink; ++= pink; +++=red; ++++= dark red or magenta.

Example 4

Employing the procedures set forth in Examples 1 and 2, and the preferred embodiment illustrated in Fig. 1, experiments were conducted adding different final concentrations of bacteria/ml to normal urine, and proceeding to test 4 drops of the urine-diluent mixture with results as follows. The scoring of filter color intensity is described in Example 3.

| E. coli CFU/ml Added to Normal Urine | Color Intensity Of Filter Surface |
|---|---|
| 0 | 0 |
| 10,000 | 0 |
| 50,000 | + |
| 100,000 | ++ |
| 1,000,000 | +++ |
| 10,000,000 | ++++ |

Example 5

A test for determination of antibiotic susceptibility may be performed as follows: The multiple test device illustrated in Fig. 4 is placed in a vertical position. Antibiotic solutions (one drop each) are dispensed into reaction wells #1 through #8. One drop of formalin solution is dispensed into well #9 and one drop of distilled water is placed into well #10. A broth suspension of bacteria is diluted to yield approximately 100,000 bacteria/ml, and one drop of this dilution is added to each reaction well #1 through #10. The device is then incubated for three to four hours at 37 degrees C. After the incubation period, 4 drops of pH 1.5 HCl diluent are added to each reaction well. The device is placed in a horizontal position to allow travel of the fluid from the reaction well into the reservoir. After the fluid is absorbed into the filter pad, the dye solution and washing reagent are placed on each filter site as described in Examples 1 and 2 above. After completion of the staining process, the filter pad is visually examined for color development. Formalin treated site #9 manifests a pink ( + + ) color and represents the zero-hour control. Site #10 manifests a dark red ( + + + + ) color and represents the uninhibited growth control. The color intensity of each site #1 through #8 is compared to the controls for interpretation of antimicrobial susceptibility. Color intensity equal to or greater than the growth control indicates that the organism is resistant to the antibiotic under test. Color intensity equal to or less than the zero-hour control indicates that the organism is susceptible to the antibiotic.

**Claims**

1. A process for concentrating, immobilizing and staining bacteria contained within a fluid sample, comprising:
   (a) diluting the fluid sample;
   (b) adsorbing the bacteria in the fluid sample to a designated portion of a filter;
   (c) staining said adsorbed bacteria at a basic pH with a staining dye operative at a basic pH; and
   (d) diffusing free staining dye from said stained adsorbed bacteria, characterized in that in step (a) the fluid sample is diluted with an inorganic acid; in step (b) the bacteria in the fluid sample is electrostatically adsorbed to the filter having a net negative charge and in step (d) the free staining dye is diffused from said stained electrostatically adsorbed bacteria to a region of said filter beyond said designated portion, leaving stained bacteria in said designated region for comparative quantitative analysis.

2. A process of Claim 1, wherein the inorganic acid is selected from the group consisting of hydrochloric acid, nitric acid and sulfuric acid and has a pH in the range of from 1 to 3.

3. A process of either Claim 1 or Claim 2, wherein said staining dye is a bacteria staining dye from a group including safranin-O and basic fuchsin operative in a pH range of from about 8 to 12 and soluble in buffers at said pH range, said staining dye being in a concentration range of from about 0.1% to about 0.001% and solubilized in a buffer from the group consisting of potassium borate, potassium carbonate and potassium hydroxide at a pH range of from about 8 to about 12.

4. A process of any one of Claims 1-3, wherein said diffusing in step (d) is accomplished by washing said stained adsorbed bacteria with a washing reagent composed of an inorganic acid from a group consisting of hydrochloric acid, nitric acid and sulfuric acid in a pH range of from about 2 to about 4.

5. A process of any one of Claims 1-4, wherein said fluid sample is urine and said process includes:
   (a) diluting said urine with an inorganic acid diluent in the pH range of from about 1 to about 3 to solubilize precipitates present in said urine, the composition of the urine-diluent mixture being in the range of from about 4 parts of diluent to about 1 part urine;
   (b) depositing said urine diluent mixture onto the surface of said filter;
   (c) allowing said urine diluent mixture to absorb into said filter; and
   (d) said diffusing comprising adding a washing reagent composed of an inorganic acid in the pH range of from about 2 to about 4 and from a group consisting of hydrochloric acid, nitric acid and sulfuric acid onto the site on said filter of the initial deposit of said urine sample so that the concentrated and stained bacteria are manifest at said site and any free dye deposited at said site with said stained bacteria is diffused remote and distal of said site in the form of a ring of free dye in the range of from about 10 mm to about 30 mm from the concentrated stained bacteria at said site.

6. A process of any one of the preceding claims, wherein said stained and adsorbed bacteria is quantitated by comparing the color intensity of said stained bacteria on said filter to a color guide having colors representing the results of tests from samples having known concentrations of bacteria.

7. An apparatus for concentrating, immobilizing and staining bacteria from a fluid sample according to the process of any one of Claims 1 to 6, comprising:
(a) disk means (1, 10) having upper and lower surfaces and an orifice (3);
(b) filter means (5);
(c) means (6, 8) retaining said filter means (5) against said lower surface of said disk means (1, 10) and means for color comparison indicating quantitation of said stained bacteria, characterized in that the disk means in (a) form a sample reservoir means (2) extending from said upper surface and opening through said orifice for conducting, in use, the fluid sample to said lower surface; the filter means in (b) has a net negative charge; and in (c) a portion of said filter means (5) is exposed to said sample reservoir means (2) at said orifice, said filter means (5) extending radially from said opening a sufficient distance in order to, in use, permit radial diffusion of free dye from said portion of the filter means (5) exposed at said orifice and leave concentrated and stained bacteria from said fluid sample at said exposed portion of said filter means (5).

8. An apparatus of Claim 7, wherein:
(a) said sample reservoir (2) is of upwardly diverging form and defines a sufficient volume for receiving a predetermined volume of said fluid sample, disk means (1, 10) defining lip means (4) about said orifice (3) at the intersection of said orifice (3) and said lower surface, said lip means (4) engaging said filter means (5);
(b) plate means (6) for securing said filter means (5) against said lower surface; and
(c) means retaining said plate means (6) and said filter means (5) in intimate assembly with said disk means (1).

9. An apparatus of either Claim 7 or Claim 8, wherein:
(a) said disk means (1, 10) defines reaction well means (11) permitting reaction of said fluid sample prior to deposit of said fluid sample into said reservoir means (2), upon substantially horizontal positioning of said disk means (1, 10) said fluid sample gravitating from said reaction well means (11) into said sample reservoir means (2).

10. An apparatus of any one of Claims 7-9 wherein:
(a) said disk means (1, 10) defines a plurality of spaced sample reservoirs (2) and a plurality of reaction wells (11) each communicating with respective sample reservoirs (2); and
(b) said disk means (1, 10) defines base means (12) orienting said disk means (1, 10) such that fluid samples in said plurality of reaction wells (11) is prevented from entering said sample reservoirs (11), upon substantially horizontal positioning of said disk means (1, 10) said fluid samples gravitating from said reaction wells (11) into respective sample reservoirs (2).

11. An apparatus of any one of Claims 7-10, wherein said retaining means (6, 8) comprises a layer of material covering said filter means (5) and having adhesive peripheral portions there of engaging said lower surface and retaining said filter means (5) in encapsulated assembly with said disk means (1, 10).

**Patentansprüche**

1. Verfahren zum Konzentrieren, Immobilisieren und Einfärben von Bakterien, die in einer Flüssigkeitsprobe enthalten sind, wobei:
(a) die Flüssigkeitsprobe mit Wasser verdünnt wird;
(b) die Bakterien in der Flüssigkeitsprobe einem bestimmten Teil eines Filters adsorbiert werden;
(c) die adsorbierten Bakterien mit einem bei einem basischen pH-Wert wirksamen Farbstoff eingefärbt werden; und
(d) der freie Einfärbe-Farbstoff von den eingefärbten adsorbierten Bakterien diffundiert wird, **dadurch gekennzeichnet,** daß im Schritt (a) die Flüssigkeitsprobe mit einer anorganischen Säure verdünnt wird; daß im Schritt (b) die Bakterien in der Flüssigkeitsprobe elektrostatisch einem Filter adsorbiert werden, das eine rein negative Charge hat, daß im Schritt (d) der frei färbende Farbstoff von den eingefärbten elektrostatisch adsorbierten Bakterien zu einem Bereich des Filters unterhalb des genannten bestimmten Bereiches diffundiert wird, und daß die eingefärbten Bakterien in dem bestimmten Bereich für eine quantitative Vergleichsanalyse belassen werden.

**2.** Verfahren nach Anspruch 1, bei dem die anorganische Säure aus einer Gruppe ausgewählt wird, die Chlorwasserstoffsäure, Salpetersäure und Schwefeldioxydsäure enthält und einen pH-Wert im Bereich von 1 bis 3 aufweist.

**3.** Verfahren nach Anspruch 1 oder 2, bei dem der Einfärbe-Farbstoff ein Bakterien-Einfärbe-Farbstoff aus einer Gruppe ist, die Safranin-O und basisches Fochin enthält, das in einem pH-Bereich von etwa 8 bis 12 wirksam und in diesem pH-Bereich in Buffern lösbar ist, daß der Einfärbe-Farbstoff in einem Konzentrations-Bereich von etwa 0,1 % bis etwa 0,001 % liegt und in einem Buffer aus der Gruppe löslich gemacht wird, die aus Kalium-Borat, Kalium-Karbonat und Kalium-Hydroxid bei einem pH-Bereich von etwa 8 bis etwa 12 besteht.

**4.** Verfahren nach einem der Ansprüche 1 - 3, bei dem die Diffusion im Schritt (d) dadurch begleitet wird, daß die eingefärbten und adsorbierten Bakterien mit einer Wasch-Reagenz gewaschen werden, die aus einer anorganischen Säure aus einer Gruppe zusammengesetzt ist, die aus Chlorwasserstoffsäure, Salpetersäure und Schwefeldioxydsäure bei einem pH-Bereich von etwa 2 bis etwa 4 besteht.

**5.** Verfahren nach einem der Ansprüche 1 - 4, bei dem die Flüssigkeitsprobe Urin ist und das Verfahren folgende Schritte enthält:
(a) Verdünnen des Urins mit einem Verdünnungsmittel aus anorganischer Säure in einem pH-Bereich von etwa 1 bis etwa 3, um in dem Urin vorhandene Abscheidungen löslich zu machen, wobei die Zusammensetzung der Urin-verdünnten Mixtur in einem Bereich von etwa vier Teilen Verdünnungsmittel zu etwa einem Teil Urin liegt;
(b) Ablage der Urin-verdünnten Mixtur auf der Oberfläche des Filters;
(c) Schaffung der Möglichkeit, daß die Urin-verdünnte Mixtur in das Filter adsorbiert wird und
(d) Zusatz einer Wasch-Reagenz, zu dem Diffussionsvorgang, die zusammengesetzt ist aus einer anorganischen Säure in einem pH-Bereich von etwa 2 bis etwa 4 aus einer Gruppe bestehend aus Chlorwasserstoffsäure, Salpetersäure und Schwefeldioxydsäure auf den Stellen des Filters der anfänglichen Niederlage der Urinprobe, so daß die konzentrierten und eingefärbten Bakterien an dieser Stelle manifastiert werden und jeder freie an dieser Stelle mit den eingefärbten Bakterien niedergelegte Farbstoff entfernt von dieser Stelle in der Form eines Ringes freien Farbstoffes im Bereich von etwa 10 mm bis etwa 30 mm von den konzentrierten, eingefärbten Bakterien an dieser Stelle diffundiert wird.

**6.** Verfahren nach einem der vorangehenden Ansprüche, wobei die genannten eingefärbten und adsorbierten Bakterien dadurch in ihrer Menge bestimmt werden, daß die Farbintensität der eingefärbten Bakterien auf dem Filter mit einer Farben aufweisenden Farbskala verglichen werden, die die Ergebnisse von Untersuchungen an Proben mit bekannten Konzentrationswerten der Bakterien aufweist.

**7.** Gerät zum Konzentrieren, Immobilisieren und Einfärben von Bakterien einer Flüssigkeitsprobe nach dem Verfahren eines der Ansprüche 1 - 6, enthaltend:
(a) eine Platte (1, 10) mit einer oberen und unteren Oberfläche und einer Ausflußöffnung (3);
(b) ein Filter (5);
(c) Mittel, (6, 8) die das Filter (5) an der unteren Oberfläche der Platte (1, 10) haltern, sowie Mittel zum Farbvergleich zur Anzeige der Menge der eingefärbten Bakterien, **dadurch gekennzeichnet,** daß die Platte im Merkmal (a) einen Behälter (2) für die Probe bildet, der sich von der oberen Oberfläche aus erstreckt und über die Ausflußöffnung offen ist und bei Gebrauch die Flüssigkeitsprobe zu der unteren Oberfläche führt; daß das Filter im Merkmal (b) eine rein negative Charge hat; und daß im Merkmal (c) ein Teil des Filters (5) gegenüber dem Probenbehälter (2) und der Ausflußöffnung offen ist, daß sich das Filter (5) radial von der Öffnung über eine ausreichende Strecke erstreckt, um beim Gebrauch eine radiale Diffusion des freien Farbstoffes von dem genannten Bereich des zu der Ausflußöffnung hin offenen Filters (5) und einen Austritt der konzentrierten und eingefärbten Bakterien aus der Flüssigkeitsprobe an der offenen Stelle des Filters (5) zu ermöglichen.

**8.** Gerät nach Anspruch 7, bei dem:
(a) der Probenbehälter (2) eine nach oben auseinandergehende Form hat und ein ausreichendes Volumen aufweist, um ein vorbestimmtes Volumen der Flüssigkeitsprobe aufzunehmen, daß die Platte (1, 10) um die Ausflußöffnung (3) herum an der Verbindungsstelle der Ausflußöffnung (3) und der unteren Oberfläche eine Lippe (4) aufweist und die Lippe (4) das Filter (5) aufnimmt;

(b) eine Platte (6), um das Filter (5) an der unterren Oberfläche zu haltern; und

(c) Mittel, die die Platte (6) und das Filter (5) in einer engen Anordnung mit der Platte (1) halten.

**9.** Gerät nach Anspruch 7 oder 8, bei dem:

(a) die Platte (1, 10) einen Reaktionsschacht (11) bildet, der eine Reaktion der Flüssigkeitsprobe ermöglicht, bevor die Flüssigkeitsprobe in dem Behälter (2) niedergelegt wird, wobei durch eine im wesentliche horizontale Positionierung der Platte (1, 10) die Flüssigkeitsprobe von dem Reaktionsschacht im den Proben-Behälter (2) durch Schwerkraft absinkt.

**10.** Gerät nach einem der Ansprüche 7 - 9, bei dem:

(a) die Platte (1, 10) eine Vielzahl von getrennten Probenbehältern (2) und eine Vielzahl von Reaktionsschächten (11) bildet, von denen jeder mit einem entsprechenden Probenbehälter (2) zusammenarbeitet; und

(b) die Platte (1, 10) ein Grundgestell (12) bildet, von dem die Platte (1, 10) derart ausgeht, daß die Flüssigkeitsproben in der Vielzahl von Reaktionsschächten (11) daran gehindert sind, in die Probenbehälter (11) einzudringen, und daß durch eine im wensentlichen horizontale Positionierung der Platte (1, 10) die Flüssigkeitsproben durch Schwerkraft von den Reaktions-Schächten (11) in die entsprechenden Probenbehälter (2) absinken.

**11.** Gerät nach einem der Ansprüche 7 - 10, worin die Haltemittel (6, 8) eine Schicht aus einem Material enthalten, das das Filter (5) abdeckt und klebende periphere Teile aufweist, die mit der unteren Oberfläche in Verbindung stehen und das Filter (5) in einer eingekapselten Anordnung mit der Platte (1, 10) halten.

**Revendications**

**1.** Un procédé de concentration, d'immobilisation et de coloration ou colorisation d'une bactérie contenue dans un échantillon de fluide comprenant les étapes consistant à:

(a) diluer l'échantillon de fluide;

(b) adsorber la bactérie dans l'échantillon de fluide sur une partie donnée d'un filtre;

(c) colorer ladite bactérie adsorbée à un pH basique, avec un pigment fonctionnant dans un pigment au pH basique; et

(d) faire diffuser le pigment libre hors de la bactérie adsorbée colorée;

caractérisé en ce que,

dans l'étape (a), l'échantillon de fluide est dilué avec un acide inorganique;

et en ce que,

dans l'étape (b), la bactérie dans l'échantillon de fluide est électrostatiquement adsorbée sur un filtre présentant une charge négative nette,

et en ce que,

dans l'étape (d), le pigment libre est diffusé hors du champ des bactéries adsorbées électrostatiquement et colorées vers une région dudit filtre s'étendant au-delà de la portion donnée, ceci laissant la bactérie colorée dans ladite région donnée, pour permettre une analyse comparative quantitative.

**2.** Un procédé selon la revendication 1, dans lequel l'acide inorganique est choisi dans le groupe comprenant l'acide chlorhydrique, l'acide nitrique, l'acide sulfurique, et dans lequel ledit acide inorganique présente un pH dans la plage de 1 à 3.

**3.** Un procédé selon la revendication 1 ou 2, dans lequel ledit pigment de colorisation est un pigment de colorisation de bactéries provenant d'un groupe comprenant la safranin-O et la Fuchsine basique, efficaces dans un domaine de pH compris entre environ 8 et environ 12, et soluble dans des tampons dans ledit domaine de pH, ledit pigment de colorisation étant dans une plage de concentration comprise entre environ 0,1% à 0,001%, et étant solubilisé dans un tampon d'un groupe consistant en borate de potassium, carbonate de potassium et hydroxyde de potassium, dans une plage de pH entre environ 8 et environ 12.

**4.** Un procédé selon l'une quelconque des revendications 1 à 3, dans lequel ladite diffusion dons l'étape (d) est obtenue en lavant ladite bactérie colorée et adsorbée avec un réactif de lavage constitué d'un acide inorganique provenant d'un groupe consistant en l'acide chlorhydrique, l'acide nitrique et l'acide sulfurique dans un domaine de pH entre environ 2 et environ 4.

5. Un procédé selon l'une quelconque des revendications 1 à 4, dans lequel ledit échantillon de fluide est de l'urine, et dans lequel ledit procédé comprend l'étape consistant à:

(a) diluer ladite urine avec un diluant acide inorganique dans une plage de pH comprise entre environ 1 et environ 3, de façon à solubiliser les précipités présents dans ladite urine, la composition du mélange urine-diluant étant dans la plage d'environ 4 parties de diluant pour environ une partie d'urine;

(b) déposer ledit mélange urine-diluant sur la surface dudit filtre;

(c) laisser le mélange urine-diluant s'absorber dans ledit filtre; et

(d) ladite étape de diffusion comprenant l'ajout d'un réactif de lavage composé d'un acide inorganique dans un domaine de pH d'environ 2 à environ 4, et provenant d'un groupe consistant dons l'acide chlorhydrique, l'acide nitrique et l'acide sulfurique, sur le site dudit filtre à l'endroit du dépôt initial dudit échantillon d'urine, de sorte que les bactéries concentrées et colorées se manifestent sur ledit site et de sorte que tout pigment libre déposé sur ledit site avec lesdites bactéries colorées soit diffusé à l'écart à l'extrémité distale dudit site, sous forme d'un anneau de pigment libre, dans une plage d'environ 10 à 30 mm de la bactérie concentrée colorée présente sur ledit site.

6. Un procédé selon l'une quelconque des revendications précédentes, dans lequel ladite bactérie colorée et adsorbée est quantifiée en comparant l'intensité de la couleur de ladite bactérie colorée sur ledit filtre à un guide de couleur présentant des couleurs représentatives des résultats d'essais provenant d'échantillons présentant des concentrations connues de bactéries.

7. Dispositif pour la concentration, l'immobilisation et la colorisation de bactéries provenant d'un échantillon de fluide selon le procédé décrit dans l'une quelconque des revendications 1 à 6, comprenant:

(a) des moyens de disque (1, 10) présentant des surfaces supérieure et inférieure et un orifice (3);

(b) des moyens de filtre (5);

(c) des moyens (6, 8) maintenant lesdits moyens de filtre (5) contre ladite surface inférieure desdits moyens de disque (1, 10), et des moyens de comparaison de couleur indiquant la quantité desdites bactéries colorées,

caractérisé en ce que

lesdits moyens de disque de (a) forment des moyens de réservoir d'échantillon (2) s'étendant à partir de ladite surface supérieure et débouchant à travers ledit orifice de façon à conduire, en cours d'utilisation, l'échantillon de fluide vers ladite surface inférieure;

en ce que

les moyens de filtre de (b) présentent une charge négative nette; et

en ce que

dans (c), une partie desdits moyens de filtre (5) est exposée auxdits moyens de réservoir d'échantillon (2) à l'endroit dudit orifice, lesdits moyens de filtre (5) s'étendant radialement à partir de ladite ouverture à une distance suffisante pour permettre, en cours d'utilisation, une diffusion radiale du pigment libre depuis ladite partie des moyens de filtre (5) faisant face audit orifice et de façon à laisser, concentrées et colorées, les bactéries séparées provenant dudit échantillon de fluide sur ladite partie exposée desdits moyens de filtre (5).

8. Un dispositif selon la revendication 7, dans lequel:

(a) ledit réservoir d'échantillon (2) présente une forme s'élargissant vers le haut, et définit un volume suffisant pour recevoir un volume prédéterminé d'échantillon de fluide, lesdits moyens de disque (1, 10) définissant des moyens de lèvre (4) autour dudit orifice (3) à l'intersection dudit orifice (3) et de ladite surface inférieure, lesdits moyens de lèvre (4) étant en prise avec lesdits moyens de filtre (5);

(b) des moyens de plaque (6) destinés à maintenir lesdits moyens de filtre (5) contre ladite surface inférieure; et

(c) des moyens de maintien desdits moyens de plaque (6) et desdits moyens de filtre (5) en assemblage monobloc avec lesdits moyens de disque (1).

9. Un dispositif selon la revendication 8 ou 7, dans lequel:

(a) lesdits moyens de disque définissent un puits de réaction des moyens de puits de réaction (11) permettant une réaction dudit échantillon de fluide avant le dépôt dudit échantillon de fluide dans lesdits moyens de réservoir (2), ledit échantillon de fluide s'écoulant depuis ledit moyen de fluide de réaction (11) vers lesdits moyens de réservoir à échantillons (2) lorsque lesdits moyens de disque (1, 10) sont disposés sensiblement horizontalement.

10. Un dispositif selon l'une quelconque des revendications 7 à 9, dans lequel:
(a) lesdits moyens de disque (1, 10) définissent une pluralité de réservoirs d'échantillons séparés l'un de l'autre, et une pluralité de puits de réaction (11), communiquant chacun avec l'un des divers réservoirs d'échantillons (2); et
(b) lesdits moyens de disque (1, 10) définissent des moyens de base (12) orientant lesdits moyens de disque (1, 10) de sorte que lesdits échantillons de fluide dans chacun desdits puits de réaction (11) soient empêchés d'entrer dans lesdits réservoirs d'échantillons (11) de sorte que lesdits échantillons de fluide s'écoulent en provenant desdits puits de réaction (11) vers lesdits réservoirs d'échantillons respectifs (2) lorsque lesdits moyens de disque (1, 10) sont disposés sensiblement horizontalement.

11. Un dispositif selon l'une quelconque des revendications 7 à 10, dans lequel lesdits moyens de fixation (6, 8) comprennent une couche de matériau couvrant lesdits moyens de filtre (5) et présentant des portions périphériques adhésives en prise avec ladite surface inférieure et fixant lesdits moyens de filtre (5) dans un assemblage d'encapsulation dans lesdits moyens de disque (1, 10).

FIG.1

FIG.2

FIG.3

FIG.4

FIG.5

FIG.6

EP 0 288 621 B1